# EUROPEAN PATENT SPECIFICATION

(11) **EP 1 004 591 B1**
(45) Date of publication and mention of the grant of the patent: **09.04.2003**
(21) Application number: 98309583.7
(22) Date of filing: 24.11.1998
(51) Int. Cl.: C07H 17/08

(54) **Process for preparing roxithromycin and derivatives thereof**
Verfahren zur Herstellung von Roxithromycin und Derivate davon
Procédé pour la préparation de roxithromycin et ses dérivés

(43) Date of publication of application: 31.05.2000
(73) Proprietor: Max India Limited, Nawanshahr, Punjab 144 553 (IN)
(72) Inventor: Murali, Madala Krishna, Kuvempunagar, Mysore - 570 023 (IN); Babu, Meduri Suresh, c/o Mr. Thammanna Gowda, Nanjangud, 571 301 Mysore (IN); Vyas, Ketan Dhansukhal, Kuvempunagar, Mysore - 570 023 (IN); Kulkarni, Ashok Krishna, Kuvempunagar, Mysore - 570 023 (IN)
(74) Representative: Davies, Jonathan Mark

(56) References cited:
- EP-A- 0 284 203
- US-A- 4 349 545
- DATABASE WPI Section Ch, Week 9326 Derwent Publications Ltd., London, GB; Class B03, AN 93-207691 XP002098434 & ES 2 036 472 A (PRODESFARMA SA) , 16 May 1993
- DATABASE WPI Section Ch, Week 9212 Derwent Publications Ltd., London, GB; Class B03, AN 92-090680 XP002098435 & ES 2 024 371 A (PRODESFARMA SA) , 16 February 1992

## Description

The present invention relates to an improved process for preparing erythromycin derivatives, in particular that known as roxithromycin, from the corresponding oxime.

Erythromycin derivatives, in particular those of general formula (1) (defined below) disclosed in US patent specification no 4 349 545, include compounds having antibacterial use. One such compound is known as roxithromycin, namely, 9-(2',5'-dioxahexyloxyimino)erythromycin or 9-[O-[(2-methoxyethoxy)methyl]oxime of erythromycin, which is a compound of formula (1) wherein R is -O(CH₂)₂-O-CH₃, A is -CH₂, and Rₐ is H: 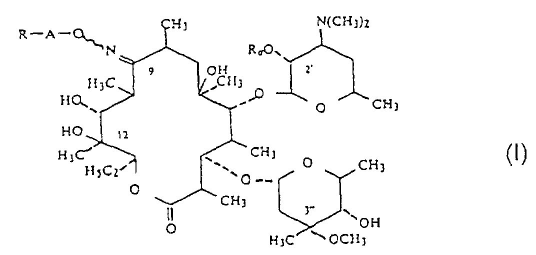 wherein A is a linear or branched alkylene of 1 to 6 carbon atoms;
R is selected from the group consisting of optionally substituted alkoxy of 1 to 6 carbon atoms, optionally substituted alkenyloxy and alkynyloxy of 2 to 6 carbon atoms, optionally substituted alkylthio of 1 to 6 carbon atoms, optionally substituted alkenylthio and alkynylthio of 2 to 6 carbon atoms with the thio groups optionally oxidized to the sulfoxide or sulfone form, optionally substituted aryloxy, and arylthio, optionally substituted aralkyloxy, and arylalkylthio, the thio derivatives optionally oxidized to sulfoxide or sulfone, -NR₁R₂, optionally substituted quaternary ammonium group, halogen, optionally substituted 1,2-epoxyethyl and the group resulting from opening of the epoxy with a nucleophilic reactant, -OOCB, a free or protected formyl, -COOR', thiocyanate, -CN, acyl and carbamoyl, R₁ and R₂ are individually selected from the group consisting of hydrogen and optionally substituted alkyl of 1 to 6 carbon atoms or taken together with the nitrogen atom to which they are attached form an optionally substituted, optionally unsaturated heterocycle which can contain another heteroatom, B is selected from the group consisting of optionally substituted alkyl and alkoxy of 1 to 6 carbon atoms, optionally substituted aryl and aryloxy and optionally substituted aralkyl and aralkoxy of 1 to 6 alkyl carbon atoms, R' is selected from the group consisting of hydrogen, a cation and an ester group; and
Rₐ is selected from the group consisting of hydrogen and acyl of an organic carboxylic acid of 1 to 18 carbon atoms;
and their non-toxic, pharmaceutically acceptable acid addition salts.

The aforementioned patent specification discloses, *inter alia*, the preparation of roxithromycin and its analogues by reacting the corresponding 9-oxime of erythromycin with a compound of formula Hal-A-R (wherein A and R as defined for the erythromycin derivative and Hal is a halogen) optionally in the presence of a base. Such bases are stated to be triethylamine; an alkali metal or alkaline earth metal carbonate or bicarbonate; or an alkali metal hydride. The reaction is preferably effected in a polar solvent at from room temperature up to the reflux temperature of the solvent. This is a bi-phasic (liquid/solid) reaction.

However, the disadvantage of such a method is that, besides in practice requiring heating, the reaction time is said to vary from several hours up to several days to go to completion. In practice, according to example 6 of the aforementioned patent specification, a first reaction period of sixteen hours is followed by two further reaction periods of fifteen and eighteen hours, respectively.

Attempts have been made by various workers in this field to provide a more economic and less time-consuming means of carrying out the reaction. Accordingly, Austrian patent specification no 94/151 discloses, *inter alia*, the conversion of the erythromycin oxime to roxithromycin and its analogues using sodium or potassium carbonate in n-butanol or acetone at a temperature of 50-120°C. Hence, significantly elevated temperatures are still required.
An alternative method has been disclosed in Spanish patent specification no 2 036 472, but this requires the use of an ammonium salt instead of the halide to react with the oxime in a non-alcoholic polar solvent. This is, again, a biphasic (liquid/solid) reaction. On the other hand, Spanish patent specifications nos 2 026 824 and 2 024 371 rely on the use of sodium or potassium hydroxide in the presence of a phase transfer catalyst in another biphasic reaction involving two immiscible liquids.

As well as long reaction times, these known processes involve controlled addition (lot-wise), elaborate work-up and several crystallisations, which makes more difficult the control of large batch production and standardisation of the process.

However, we have surprisingly found that the use of sodium methoxide as the base in the reaction enables the reaction to proceed more quickly and at a lower temperature than the prior art methods using metal bicarbonates, carbonates or hydroxides.

Accordingly, the present invention provides a process for the preparation of a compound of formula (I), as defined hereinabove, which process comprises reacting a 9-oxime of erythromycin of formula (II): 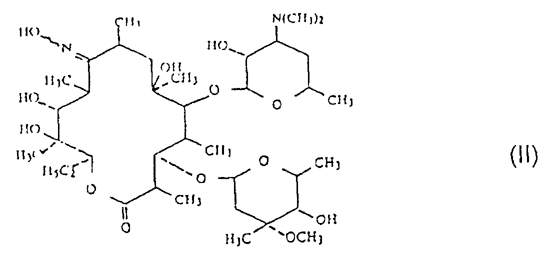 with a compound of formula (III):

X-A-R (III)

wherein A and R have the same meaning as in formula (I) and X is a leaving group;
together with sodium methoxide; and,
optionally thereafter, converting the compound of formula (I) so prepared to any other desired compound of formula (I) or salt thereof.

Preferably, the reaction is carried out in a single-phase system, particularly, a liquid single-phase system in which the reactants are both in the liquid phase and are mutually miscible.

Preferably, the process is carried out in the presence of an organic, more preferably a polar organic, solvent such as a non-alcoholic solvent. Conveniently, the solvent may be chosen from polar aprotic solvents and halogenated alkanes, ketones, nitriles, esters of organic acids, dimethylformamide (DMF), dimethylsulphoxide, hexamethylphosphotriamide, and ethers such as dialkylethers, tetrahydrofuran and dioxane, and mixtures of these. Especially convenient solvents are those having a low number of carbon atoms in the alkyl chain, such as from 1 to 4, such as acetone, acetonitrile, ethylacetate, diethylether, methylene dichloride, ethylene dichloride and chloroform, and the like. Particularly preferred solvents for use in accordance with the invention are DMF and ethylacetate, especially DMF.

Since one of the advantages of the process according to the present invention is its ability to go to completion in the absence of significantly elevated temperatures, it will be evident that preferred temperatures are as low as possible and do not exceed 50°C. More preferred is when the reaction is carried out at a temperature in the range of from -10-40°C, such as -10-30°C, especially preferred is below room or ambient temperature, such as less than about 20-22°C, more especially in the range of from 0 to 10°C.

Depending upon the precise reagents and reaction conditions chosen, especially the temperature, the reaction according to the present invention can surprisingly go to completion in a few hours, such as in up to 12 hours, more usually in up to 8 hours, such as in the range of from 2 to 6 hours, preferably from 2 to 3 hours.

Suitable compounds of formula (III) include those wherein X is chosen from any of the standard leaving groups known to those skilled in the art. Preferred such leaving groups include those suitable for nucleophilic substitution; especially preferred is halo, such as chloro. A particularly preferred compound of formula (III) is (methoxyethoxy)methyl chloride (MEM.CI).

Therefore, the invention accordingly provides a process for the preparation of roxithromycin, which process comprises reacting erythromycin 9-oxime with methoxyethoxymethyl-X (where X is as defined hereinabove, especially chloride) and sodium methoxide in the presence of an aprotic polar or halogenated solvent at a temperature below 50°C, such as at -10-40°C, for less than 12 hours, such as for 2-6 hours. In particular, the present invention provides such a reaction carried out in a single, liquid/liquid phase.

According to a more detailed description of the preferred process of this invention, an erythromycin A oxime (more preferably, 1.0 mole) solution in the organic solvent (more preferably, in a ratio of oxime:solvent in the range of from 1: 2.0 to 4.5) is mixed with the sodium methoxide (more preferably, 1.1-1.8 moles). Controlled addition of (methoxyethoxy) methyl halide(s) (more preferably 1.0-1.5 mole) over a period of time of about 2 to 6 h, preferably 2-3h, completes the etherification of the oxygen function in the oxime. Then the organic phase is preferably washed with water or saturated sodium chloride solution and, after complete removal of solvent, the residue can be crystallised, such as from methanol/water or methanol, to get pure roxithromycin directly. In the case of a water-miscible solvent, the work-up will change from removal of the solvent to simply precipitating the product in crude form from the reaction mass by adding water. The precipitate so obtained can then be separated by filtration and crystallised, for example, from methanol/water or methanol.

The process of the present invention, besides also affording the advantage of directly yielding the final product in a substantially pure form, also permits the use of less base and less MEM.Cl in comparison with the prior art processes.

Also, since the etherification reaction in this process proceeds faster than in the prior art processes, the time the erythromycin A oxime or roxithromycin formed remains in the solution phase is significantly less than in the prior art processes. Hence, there is less decomposition, which helps this process to result in nearly quantitative yields.

Furthermore, the process of the present invention has the advantage of permitting large batch production and,enables control on stabilisation of yields & reproducibility, with improved time cycles.

The invention therefore further provides the use of sodium methoxide in the preparation of an erythromycin derivative, such as one hereinbefore described, in particular, roxithromycin, in particular, in a single phase reaction.

The compound of formula (I), such as roxithromycin, thus prepared, may then be formulated, for example, by bringing it into association with a suitable carrier therefor, into a pharmaceutical formulation, as described in the aforementioned patent specifications or otherwise as known to those skilled in the art.

The present invention will now be illustrated by the following example(s).

### EXAMPLE 1: Preparation of Roxithromycin using Sodium Methoxide /Ethyl Acetate

Erythromycin A oxime (37.5 g, 0.05 mole) is dissolved in ethyl acetate (115 ml), cooled to 0-5°C and sodium methoxide (3.24 g, 0.06 mole) added. A solution of (methoxyethoxy)methyl chloride (6.85 g, 0.055 mole) dissolved in ethyl acetate (20 ml) is slowly added to the above cooled solution with stirring over 2-3 h at 0-5°C. The reaction mixture is stirred for 30 min. and completion of the reaction monitored by TLC for the absence of erythromycin A oxime.

The reaction mixture temperature is then raised to ambient, and washed with water and 10% NaCI solution. The organic phase is dried over anhydrous MgSO₄ and the solvent is completely removed under vacuum at 50°C. The residue is dissolved in 50 ml of hot methanol, treated with 2.0 g activated charcoal, filtered and washed on a carbon bed with 10 ml hot (50°C) methanol. The filtrate is gradually cooled to 30°C and the solution stirred for 6-8 h at the same temperature. The crystallised roxithromycin is collected by filtration, and washed and dried at 50-55°C to obtain a first yield of 25 g (m.p. 119 - 120°C) and a second yield of 12.5 g.

### EXAMPLE 2: Preparation of Roxithromycin using Sodium Methoxide /DMF

Erythromycin A oxime (37.5 g, 0.05 mole) is dissolved in dimethyl formamide (DMF) (100 ml) and cooled to 0-5°C. Sodium methoxide (3.24 g, 0.062 mole) is added followed by (methoxyethoxy)methyl chloride (6.85 g, 0.055 mole) dissolved in DMF (12.5 ml), slowly with stirring, over 2-3 hours at 0-5°C. The reaction is monitored by TLC until erythromycin A oxime disappears.
Then the reaction mixture temperature is raised to ambient and, water (350ml) added over 1 hour. The slurry is stirred for 2 hours, then the crystalline precipitate is collected by filtration and thoroughly washed with water (200 ml).

The resulting wet, crude roxithromycin (65 g) is taken up in methanol (75 ml), dissolved by heating and decolourised with activated carbon (2.0 g). The filtrate is cooled slowly to 30°C and maintained for 6-8 hours. The crystalline product is collected by filtration, and washed and dried at 55°C. The first yield is 30.0 g (m.p. 119-120°C), and the second yield 7.0 g.

### EXAMPLE 3: Preparation of Roxithromycin using Sodium Methoxide /CH₂ Cl₂

Erythromycin A oxime (37.5 g, 0.05 mole) is dissolved in methylene dichloride (337.5 ml), cooled to 0-5°C and sodium methoxide (3.24 g, 0.06 mole) added. A solution of (methoxyethoxy)methyl chloride (6.85 g, 0.055 mole) dissolved in methylene dichloride (37.5 ml) is slowly added to the above cooled solution under stirring over 2-3 h at 0-5°C. The reaction mixture stirred for 30 min. and the end of the reaction monitored by TLC for the absence of erythromycin A oxime.

The reaction mixture temperature is then raised to ambient, and washed with water and saturated NaCl solution. The organic phase is dried over anhydrous MgSO₄ for 30 min. and the solvent is completely removed under vacuum at 40°C. The residue is dissolved in methanol (50 ml), treated with activated charcoal (2.0 g), and filtered and washed on a carbon bed with hot methanol (50°C, 10 ml). The filtrate is gradually cooled to 30°C and the solution stirred for 6-8 h at the same temperature. The resulting crystallised roxithromycin is collected by filtration, and washed and dried at 50-55°C to obtain first yield of 24 g (m.p. 119-120°C), and a second yield of 11.0 g.

## Claims

1. A process for the preparation of a compound of formula (I): 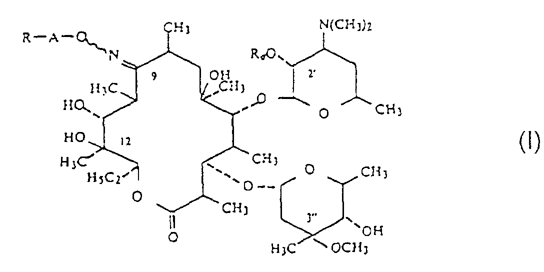 wherein A is a linear or branched alkylene of 1 to 6 carbon atoms;
R is selected from the group consisting of optionally substituted aikoxy of 1 to 6 carbon atoms, optionally substituted alkenyloxy and alkynyloxy of 2 to 6 carbon atoms, optionally substituted alkylthio of 1 to 6 carbon atoms, optionally substituted alkenylthio and alkynylthio of 2 to 6 carbon atoms with the thio groups optionally oxidized to the sulfoxide or sulfone form, optionally substituted aryloxy, and arylthio, optionally substituted aralkyloxy and arylalkylthio, the thio derivatives optionally oxidized to sulfoxide or sulfone, -NR₁R₂, optionally substituted quaternary ammonium group, halogen, optionally substituted 1,2-epoxyethyl and the group resulting from opening of the epoxy with a nucleophilic reactant, -OOCB, a free or protected formyl, -COOR', thiocyanate, -CN, acyl and carbamoyl, R₁ and R₂ are individually selected from the group consisting of hydrogen and optionally substituted alkyl of 1 to 6 carbon atoms or taken together with the nitrogen atom to which they are attached form an optionally substituted, optionally unsaturated heterocycle which can contain another heteroatom, B is selected from the group consisting of optionally substituted alkyl and alkoxy of 1 to 6 carbon atoms, optionally substituted aryl and aryloxy and optionally substituted aralkyl and aralkoxy of 1 to 6 alkyl carbon atoms, R' is selected from the group consisting of hydrogen, a cation and an ester group; and
Rₐ is selected from the group consisting of hydrogen and acyl of an organic carboxylic acid of 1 to 18 carbon atoms;
and their non-toxic, pharmaceutically acceptable acid addition salts, which process comprises reacting a 9-oxime of an erythromycin derivative of formula (II): 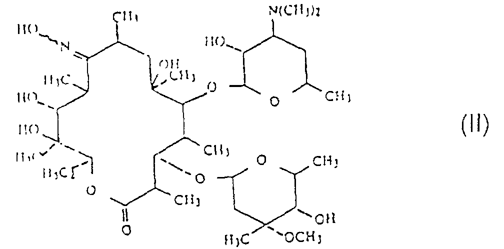 with a compound of formula (III):
X-A-R (III)
wherein A and R have the same meaning as in formula (I) and X is a leaving group;
together with sodium methoxide.

2. A process according to claim 1, further comprising, thereafter, converting the compound of formula (I) so prepared to any other desired compound of formula (I) or salt thereof.

3. A process according to claim 1 or claim 2, wherein R is -O(CH₂)₂-O-CH₃, A is -CH₂, and Rₐ is H for the preparation of roxithromycin, namely, 9-(2',5'-dioxahexyloxyimino)erythromycin or the 9-[O-[(2-methoxyethoxy)methyl]oxime of erythromycin.

4. A process according to any of claims 1 to 3, wherein the reaction is carried out in a single-phase system.

5. A process according to any preceding claim, wherein the reaction is carried out in a liquid single-phase system in which the reactants are both in the liquid phase and are mutually miscible.

6. A process according to claim 5, in which the liquid single phase is selected from polar aprotic solvents and halogenated alkanes, ketones, nitriles, esters of organic acids such as ethyl acetate, dimethylformamide, dimethylsulphoxide, hexamethylphosphotriamide, and ethers such as dialkylethers, tetrahydrofuran and dioxane, and mixtures of these.

7. A process according to any preceding claim, wherein the reaction is carried out at a temperature in the range of from -10 to 40°C.

8. A process according to claim 7, wherein the temperature is in the range of from 0 to 10°C.

9. A process according to claim 3, wherein the reaction is carried out at a temperature in the range of from 0 to 5°C to completion in the range of from 2 to 6 hours.

10. A process according to any preceding claim, wherein, in the compound of formula (III), R is-O(CH₂)₂-O-CH₃, A is - CH₂ and X is halo.

11. A process for the preparation of roxithromycin, which process comprises reacting erythromycin 9-oxime with methoxyethoxymethyl-X , wherein X is a leaving group, and sodium methoxide in the presence of an aprotic polar or halogenated solvent at a temperature below 50°C, for less than 12 hours.

## Patentansprüche

1. Verfahren zur Herstellung einer Verbindung der Formel (I) : 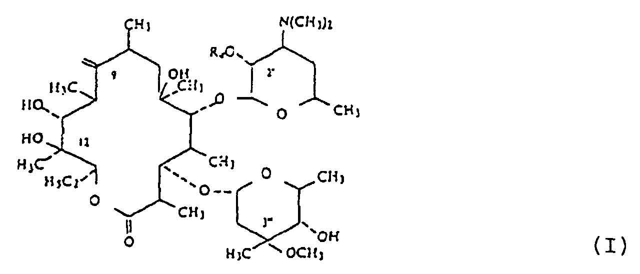 wobei A ein lineares oder verzweigtes Alkylen mit 1 bis 6 Kohlenstoffatomen ist; R ausgewählt ist aus der Gruppe bestehend aus optional substituiertem Alkoxy mit 1 bis 6 Kohlenstoffatomen, optional substituiertem Alkenyloxy und Alkynyloxy mit 2 bis 6 Kohlenstoffatomen, optional substituiertem Alkylthio mit 1 bis 6 Kohlenstoffatomen, optional substituiertem Alkenylthio und Alkynylthio mit 2 bis 6 Kohlenstoffatomen, wobei die Thio-Gruppen optional zur Sulfoxid- oder Sulfonform oxydiert sind, optional substituiertem Aryloxy, und Arylthio, optional substituiertem Aralkyloxy und Arylalkylthio, wobei die Thio-Derivate optional zu Sulfoxid oder Sulfon oxydiert sind, -NR₁R₂, optional substituierter quartärer Ammoniumgruppe, Halogen, optional substituiertem 1,2-Epoxyethyl und der Gruppe, die aus dem Öffnen des Epoxids mit einem nucleophilen Reaktanten resultiert, -OOCB, einem freien oder geschützten Formyl, -COOR', Thiocyanat, -CN, Acyl und Carbamoyl, R₁ und R₂ individuell ausgewählt sind aus der Gruppe bestehend aus Wasserstoff und optional substituiertem Alkyl mit 1 bis 6 Kohlenstoffatomen oder zusammen mit dem Stickstoffatom, an das sie gebunden sind, einen optional substituierten, optional ungesättigten Heterocyclus bilden, der ein anderes Heteroatom enthalten kann, B ausgewählt ist aus der Gruppe bestehend aus optional substituiertem Alkyl und Alkoxy mit 1 bis 6 Kohlenstoffatomen, optional substituiertem Aryl und Aryloxy und optional substituiertem Aralkyl und Aralkoxy mit 1 bis 6 Alkylkohlenstoffatomen, R' ausgewählt ist aus der Gruppe bestehend aus Wasserstoff, einem Kation und einer Estergruppe; und
Rₐ ausgewählt ist aus der Gruppe bestehend aus Wasserstoff und Acyl einer organischen Carbonsäure mit 1 bis 18 Kohlenstoffatomen;
und deren nichttoxischen, pharmazeutisch akzeptablen Säureadditionssalze,
wobei das Verfahren das Reagieren eines 9-Oxims eines Erythromycin-Derivats der Formel (II) : 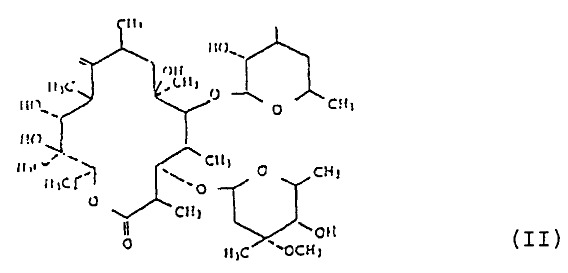 mit einer Verbindung der folgenden Formel (III) umfasst:
X-A-R (III)
wobei A und R die gleiche Bedeutung wie in der Formel (I) haben und X eine Austrittsgruppe ist;
zusammen mit Natriummethoxid.

2. Verfahren nach Anspruch 1, ferner umfassend im Anschluss daran das Umwandeln der so hergestellten Verbindung der Formel (I) in irgendeine andere erwünschte Verbindung der Formel (I) oder deren Salz.

3. Verfahren nach Anspruch 1 oder 2, wobei R -O(CH₂)₂-O-CH₃ ist, A -CH₂ ist und Rₐ H ist, zur Herstellung von Roxithromycin, nämlich 9-(2',5'-Dioxahexyloxyimino) erythromycin oder das 9-[O-[(2-Methoxyethoxy)methyl]oxim von Erythromycin.

4. Verfahren nach einem der Ansprüche 1 bis 3, wobei die Reaktion in einem Einphasensystem durchgeführt wird.

5. Verfahren nach einem der vorherigen Ansprüche, wobei die Reaktion in einem einphasigen Flüssigkeitssystem durchgeführt wird, in dem sich die Reaktanten beide in der Flüssigphase befinden und miteinander mischbar sind.

6. Verfahren nach Anspruch 5, wobei die Einphasenflüssigkeit ausgewählt ist aus polaren aprotischen Lösungsmitteln und halogenierten Alkanen, Ketonen, Nitrilen, Estern organischer Säuren wie Ethylacetat, Dimethylformamid, Dimethylsulfoxid, Hexamethylphosphotriamid und Ethern wie Dialkylether, Tetrahydrofuran und Dioxan und Gemischen davon.

7. Verfahren nach einem der vorherigen Ansprüche, wobei die Reaktion bei einer Temperatur zwischen -10 und 40°C durchgeführt wird.

8. Verfahren nach Anspruch 7, wobei die Temperatur zwischen 0 und 10°C liegt.

9. Verfahren nach Anspruch 3, wobei die Reaktion 2 bis 6 Stunden lang bei einer Temperatur zwischen 0 und 5°C bis zum Abschluss durchgeführt wird.

10. Verfahren nach einem der vorherigen Ansprüche, wobei in der Verbindung der Formel (III) R -O(CH₂)₂-O-CH₃ ist, A -CH₂ und X Halo ist.

11. Verfahren zur Herstellung von Roxithromycin, wobei das Verfahren die folgenden Schritte umfasst: Reagieren von Erythromycin-9-oxim mit Methoxyethoxymethyl-X, wobei X eine Austrittsgruppe ist, und Natriummethoxid in Anwesenheit eines aprotischen polaren oder halogenierten Lösungsmittels bei einer Temperatur unter 50°C über einen Zeitraum von weniger als 12 Stunden.

## Revendications

1. Procédé pour la préparation d'un composé de la formule (I) : 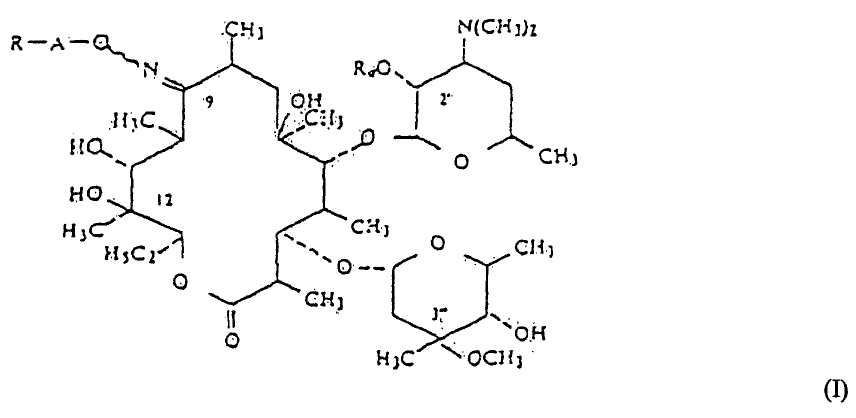 où A est un alcoylène linéaire ou ramifié de 1 à 6 atomes de carbone; R est sélectionné parmi le groupe consistant en alcoxy de 1 à 6 atomes de carbone facultativement substitué, en alcényloxy et alkynyloxy de 2 à 6 atomes de carbone facultativement substitués, en alkylthio de 1 à 6 atomes de carbone facultativement substitué, en alcénylthio et en alkynylthio de 2 à 6 atomes de carbone facultativement substitués, avec les groupes thio facultativement oxydés en la forme de sulfoxyde ou de sulfone, en aryloxy facultativement substitué, et en arylthio, en aralkyloxy et en arylalkylthio facultativement substitués, les dérivés thio facultativement oxydés en sulfoxyde ou sulfone, -NR₁R₂, en groupe ammonium quaternaire facultativement substitué, en halogène, en 1,2-époxyéthyle facultativement substitué et le groupe résultant de l'ouverture de l'époxy avec un réactif nucléophile, -OOCB, un formyl libre ou protégé, -COOR', un thiocyanate, -CN, un acyle et un carbarnoyl, R₁ et R₂ sont sélectionnés individuellement parmi le groupe consistant en hydrogène et en alkyle de 1 à 6 atomes de carbone facultativement substitué ou pris ensemble avec l'atome d'azote auquel ils sont attachés, forment un hétérocycle facultativement substitué, facultativement insaturé, qui peut contenir un autre hétéroatome, B est sélectionné parmi le groupe consistant en alkyle et alcoxy de 1 à 6 atomes de carbone facultativement substitués, en aryle et aryloxy facultativement substitués et en aralkyle et aralcoxy de 1 à 6 atomes de carbone alkylique facultativement substitués, R' est sélectionné parmi le groupe consistant en hydrogène, un cation et un groupe ester; et Rₐ est sélectionné parmi le groupe consistant en hydrogène et en acyle d'un acide carboxylique organique de 1 à 18 atomes de carbone; et leurs sels d'addition d'acides non toxiques, pharmaceutiquement acceptables, lequel procédé comprend mettre à réagir une 9-oxime d'un dérivé d'érythromycine de la formule (II): 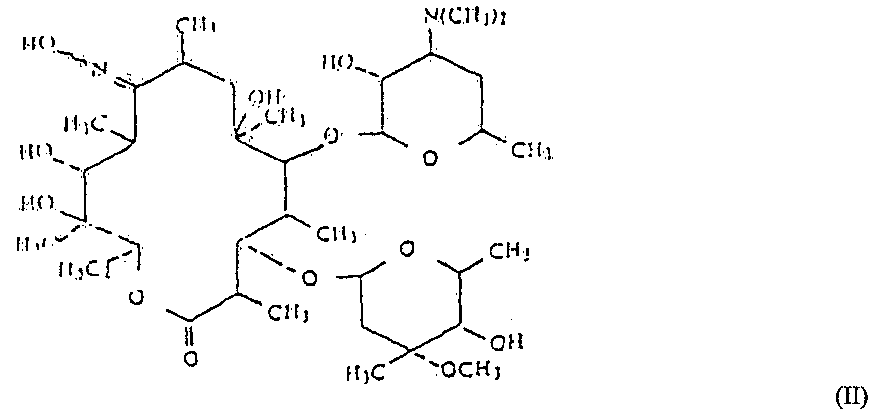 avec un composé de la formule (III) :
X-A-R (III)
où A et R ont la même signification que dans la formule (I) et X est un groupe labile; avec du méthylate de sodium.

2. Procédé selon la revendication 1, comprenant en plus convertir, ensuite, le composé de la formule (I) ainsi préparé, en n'importe quel autre composé désiré de la formule (I) ou sel de celui-ci.

3. Procédé selon la revendication 1 ou la revendication 2, où R est -O(CH₂)₂-O-CH₃, A est -CH₂, et Rₐ est H pour la préparation de roxithromycine, nommément, 9-(2',5'-dioxahexyloxyimino)érythromycine ou la 9-[O-[(2-méthoxyéthoxy)méthyl]oxime d'érythromycine.

4. Procédé selon l'une quelconque des revendications 1 à 3, dans lequel la réaction est menée à bien dans un système en phase unique.

5. Procédé selon l'une quelconque des revendications précédentes, dans lequel la réaction est menée à bien dans un système en phase unique liquide dans lequel les réactifs sont tous les deux en phase liquide et sont mutuellement miscibles.

6. Procédé selon la revendication 5, dans lequel la phase unique liquide est sélectionnée parmi des solvants polaires aprotiques et des alcanes halogénés, des cétones, des nitriles, des esters d'acides organiques tels que l'acétate d'éthyle, le diméthylformamide, le diméthyle sulfoxyde, l'hexaméthyl-phosphotriamide, et des éthers tels que les éthers dialkyliques, le tétrahydrofurane et le dioxanne, et des mélanges de ceux-ci.

7. Procédé selon l'une quelconque des revendications précédentes, dans lequel la réaction est menée à bien à une température dans la plage de -10 à 40° C.

8. Procédé selon la revendication 7, dans lequel la température est dans la plage de 0 à 10° C.

9. Procédé selon la revendication 3, dans lequel la réaction est menée à bien à une température dans la plage de 0 à 5° C jusqu'à l'achèvement dans la plage de 2 à 6 heures.

10. Procédé selon l'une quelconque des revendications précédentes, dans lequel, dans le composé de la formule (III), R est -O(CH₂)₂-O-CH₃, A est -CH₂ et X est un halogène.

11. Procédé pour la préparation de roxithromycine, lequel procédé comprend mettre à réagir de la 9-oxime d'érythromycine avec du méthoxyéthoxyméthyl-X, où X est un groupe labile, et du méthylate de sodium en présence d'un solvant aprotique polaire ou halogéné à une température inférieure à 50° C, pendant moins de 12 heures.
